# EUROPEAN PATENT SPECIFICATION

(11) **EP 4 406 584 B1**
(45) Date of publication and mention of the grant of the patent: **29.07.2026**
(21) Application number: 24181679.2
(22) Date of filing: 20.11.2019
(51) Int. Cl.: A61M 25/00, A61M 27/00

(54) **MEDICAL DEVICE PACKAGE**
GEHÄUSE FÜR MEDIZINISCHE VORRICHTUNG
EMBALLAGE DE DISPOSITIF MÉDICAL

(30) Priority: 21.11.2018 US 201862770342 P
(43) Date of publication of application: 31.07.2024
(62) Divisional of application: 19828923.3
(73) Proprietor: Hollister Incorporated, Libertyville, IL 60048 (US)
(72) Inventor: MURRAY, Michael G, Libertyville, IL 60048 (US); O'FLYNN, Padraig M., Libertyville, IL 60048 (US)
(74) Representative: FRKelly

(56) References cited:
- WO-A1-2017/001830
- GB-A- 1 440 461
- US-A- 5 184 771
- US-A1- 2012 128 835
- US-A1- 2018 021 481

## Description

The present application claims the benefit and priority of U.S. Provisional Patent Application No. 62/770,342, filed November 21, 2018.

### Field of the Disclosure

The present disclosure is generally related to medical device packaging and more particularly to a package for urinary catheters.

### Background

Intermittent catheterization is a good option for many people who suffer from various abnormalities of the urinary system. Those with such abnormalities often find it desirable to use individually-packaged, sterile catheters designed for a single use. An important criteria for such a single use product includes the cost and ease of use in performing intermittent catheterization. With regard to both cost and ease of use, these factors apply to both the catheter itself and the package for the catheter. It is desirable that end users find the package acceptable to enhance the chances of successful intermittent catheterization. In this regard an important factor in catheter package design is recognition that some catheter users will have limited manual dexterity, which can make it difficult for them to open a conventional package.

Traditionally, flow wrap catheter packages are formed with end seals running across the narrow width of the package and a fin seal running the length of the package. This type of catheter package is shown in U.S. Patent Application No. 2016/0325903. Other package designs use an opening tab that when pulled, reveals an opening in the package. This type of opening is shown in U.S. Patent Application No. 2011/0056852. In this package the entire tab is placed within the perimeter of the package which could be difficult for users with poor dexterity to grip the opening tab. WO2017001830 discloses an intermittent urinary catheterisation package including a catheter and a sealed envelope closely surrounding the catheter. US2012128835 discloses flexible film packages having a partial, initial seal against ambient atmosphere and are easily openable and reclosable. GB1440461 discloses a package formed from a thermoplastics sheet that has a longitudinal seam and intersecting transverse seams, each seam being an inset heat-seal with external fin portions and having a cut in the fin portion directed towards a corner between a longitudinal and transverse seam to partially project into one of these seams. US5184771 discloses an essentially hermetic wrapping, in particular a tubular pouch wrapping, comprising two strip-like edge zones of the wrap material that are joined together by sealing, and two incisions are present in at least one of the two said edge zones, the wrap material forming a grip tab between said two incisions by means of which a tear-open region is pulled off when the wrapping is being opened. US2018021481 discloses a urinary catheter packaging.

Therefore, there is still a need for improved easy to open catheter packaging.

### Summary of the disclosure

In one aspect the present disclosure is directed to a package formed of an elongated sheet material wrapped about a product such as a catheter. The package has a top edge, a bottom edge and opposing side edges defining a perimeter. The package also has a front panel that includes a perforation line defining an opening flap. An opening tab has a first portion that is adhered to the front panel and overlays the opening flap and a second free portion that extends beyond the perimeter of the package. The second free portion of the opening tab includes a gripping portion.

In another aspect, a catheter product including a catheter and a package containing the catheter, wherein the package is formed from a sheet material having first and second fin forming sections, first and second back forming sections, and a front forming section. The package having a top edge, a bottom edge and opposed side edges. The first and second back forming sections of the sheet material defining a back panel of the package. The front forming section of the sheet material defining a front panel of the package. The front and back panels being sealed together to form opposed side seals that extend along the opposed side edges of the package. The first and second fin forming sections of the sheet material sealed together to form a fin seal that extends between the opposed side seals and is perpendicular to a longitudinal axis of the catheter. Optionally, the fin seal may include a sealed portion and first and second free end portions wherein the first and second free end portions may be pulled apart to open the sealed portion.

### Summary of the invention

The invention is defined by independent apparatus claim 1 and further defined by the dependent claims.

### Brief Description of the Drawings

Fig. 1 is a perspective view of one embodiment of a package in accordance with the present disclosure.
Fig. 2 is a perspective view of another embodiment of a package in accordance with the present disclosure.
Fig. 3 is a top plan view of the sheet material used to form the package of Fig. 1.
Fig. 4 is a top plan view of another embodiment of a package in accordance with the present disclosure.
Fig. 5 is a side view of the package of Fig. 4.
Fig. 6 is a front perspective view of the package of Fig. 4.
Fig. 7 is a front perspective view of the package of Fig. 4 in an opened configuration.
Fig. 8 is a schematic illustration of one process for manufacturing a medical device product having the package shown in Fig. 4.

### Detailed Description of the Embodiments

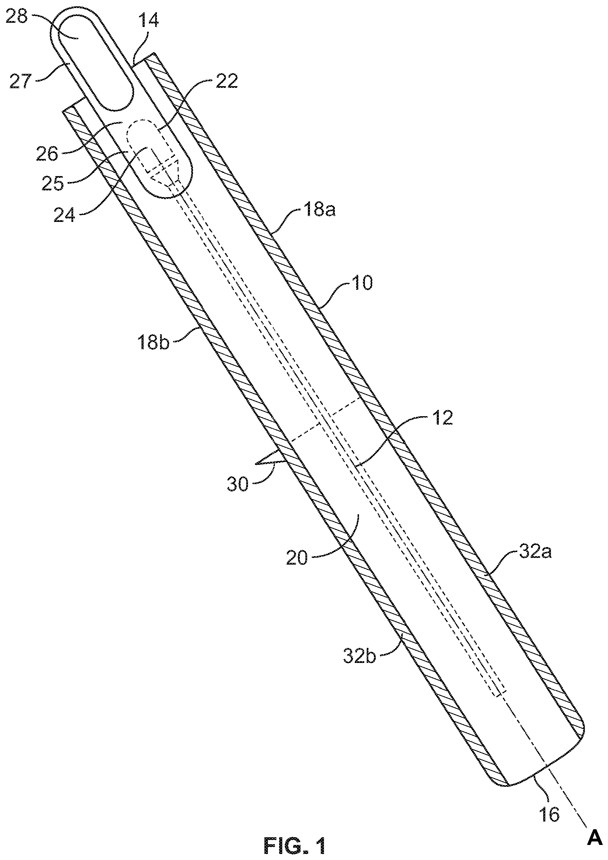

Fig. 1 shows one embodiment of a package 10. The package may be formed by wrapping an elongated sheet material around a catheter 12. The wrapped sheet includes a top edge 14, bottom edge 16 and opposed side edges 18a and 18b which define a perimeter of the package 10. The package 10 has a front panel 20 including a perforation line 22 defining an opening flap 24. An opening tab 26 has a first portion 25 that is adhered to the front panel 20 and overlies the opening flap 24. A second free portion 27 of the opening tab 26 extends beyond the perimeter of the package and has a finger hole 28 to assist with gripping the opening tab 26.

The package 10 is sealed by a fin seal 30 that runs perpendicular to the longitudinal axis A of the catheter 12 and across the package 10 between side edge seals 32a and 32b. In the illustrated package 10, the width between edge seals 32a and 32b may be shorter than the length between top edge 14 and bottom edge 15. The fin seal 30 extends in the direction of the width between edge seals 32a and 32b. As will be more fully explained below, the fin seal 30 is formed by two confronting fin panels of the wrapped sheet material, while the side edge seals are formed by adhering a first and second back panel placed in confronting relation to the front panel.

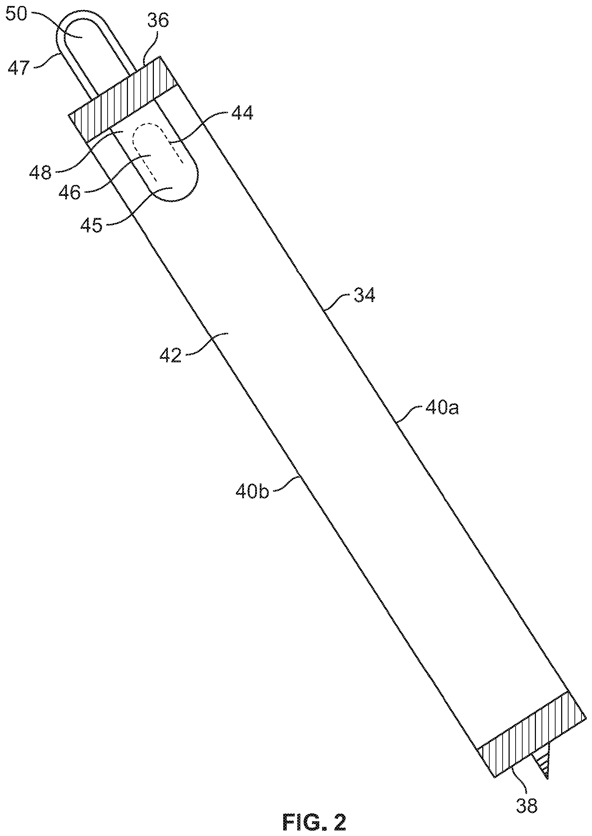

Fig 2. illustrates another embodiment of a package 34. The package 34 is formed by wrapping an elongated sheet material around a catheter. Once wrapped, the package 34 includes a top edge 36, a bottom edge 38 and opposing side edges 40a and 40b. The front panel 42 of the package 34 includes a perforation line 44 defining an opening flap 46. An opening tab 48 is adhered to the front panel 42 and has a first portion 45 that overlies the opening flap 46 and a second free portion 47 that extends beyond the perimeter of the package 34. The second free portion of the opening tab 48 that extends beyond the perimeter of the package 34 includes a finger hole 50 that assists with gripping the opening tab 48.

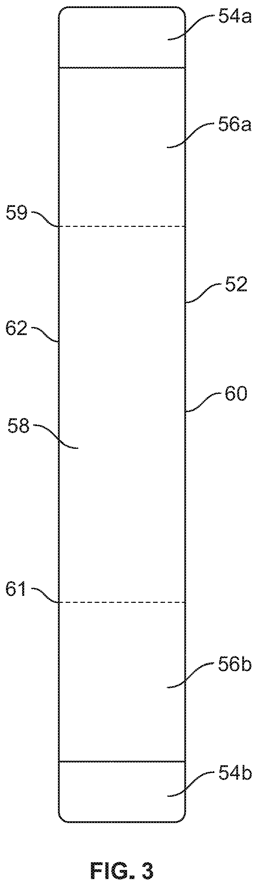

Turning now to Fig. 3, a representation of the sheet material 52 is shown prior to any forming steps. To assist in understanding how the sheet material 52 is folded and sealed, the sheet material is shown having five sections which are divided by imaginary demarcation lines. The panels may be designated as fin forming sections 54a and 54b, back forming sections 56a and 56b and front forming section 58. The sheet material 52 of course has top and bottom surfaces, which will become inside and outside surfaces of the package. All or a portion of the inside surfaces may have an adhesive coating or heat seal layer thereon. Typically this is a heat-activated adhesive that will adhere to at least other similarly coated surfaces when pressed against such surfaces and heated. Other types of adhesives could be used.

Also, while Figs. 1 and 2 show just a single package, it will be understood that the manufacturing process described below is advantageously performed in a continuous manner. That is, a continuous roll of sheet material is fed successively to a series of forming stations, (e.g., stations for wrapping, fin sealing, pinching, end sealing, punching, etc.), with individual packages not being separated from the continuous roll until they have passed through all of the stations.

It is known to form catheter packages by flow wrapping a sheet material. This process forms a package with end seals that run across the narrow width of the package and a fin that runs the entire length of the package. Formation of the package shown in Fig. 1 involves a reverse flow wrap package wherein the fin runs across the narrow width of the package and end seals run the entire length of the package.

Package formation may begin by unrolling the sheet material into a flat, generally horizontal condition with a front or leading edge 60 that will be fed to successive forming stations or zones. At a loading station, a catheter (not shown) is placed on the inside surface of the sheet material 52, at or near center of the front forming section 58. The sheet material, with the catheter on its inside surface, is then advanced to a wrapping station or zone wherein the fin forming sections 54a and 54b of the sheet 52 are wrapped or folded up and around the catheter. The inside surfaces of just the fin forming sections 54a and 54b are placed in confronting relation. This creates the three dimensional package 10.

Next the confronting fin forming sections 54a and 54b pass through a sealing station where rollers or the like create the fin seal 30. Typically the fin seal is created by heated rollers that activate the adhesive on portions of the inside surfaces of fin forming sections 54a and 54b.

Once the three dimensional body of package 10 and fin seal 30 have been formed, the next step in the manufacturing process is preparation for creation of the opposing side edge seals 32a and 32b. This is done at a pinching zone where portions of the back forming sections 56a and 56b and front forming section 58 of the sheet material 52 are folded in at the upper fold line 59 and lower fold line 61 and are placed in a generally flat condition, with the back forming sections 56a and 56b and front forming section 58 confronting one another. Since the packages are ideally made of a continuous roll of sheet material, the side edge seal of the front or leading edge 60 is formed simultaneously with the immediately-adjacent side edge seal of an end or trailing edge 62 of a successive, leading package. That is, both of the side edge seals are created at a single pinching and side edge sealing zone but the single sealing zone creates side edge seals for what will become separate packages. The leading and trailing packages are subsequently separated from one another at a final severing station. In the pinching zone, portions of the back forming sections and front forming section are pinched together into a generally horizontal condition. Also, in connection with the pinching operation the fin seal 30 is folded down into a generally horizontal condition. Folding of the fin seal may be done either before, during or after the pinching operation of the back forming sections and the front forming section.

Once pinching and folding operations are complete, the sheet enters the side edge seal station. Here hot, transverse rollers contact the pinched portions to form the end or trailing side edge seal 32b of a leading package and the front or leading side edge seal 32a of a trailing package. The side edge seals 32a and 32b are shown in Fig. 1 by the cross-hatching.

It is pointed out that since the outside surfaces of the sheet material do not have an adhesive on them, the side edge sealing operation does not result in fastening the fin seal to the outside surface of the package. After side edge sealing the fin 30 is free to fold back up, out of the plane of the side edge seals, as shown by Fig. 1.

While the package is held taught during the end sealing operation, it is an advantageous time to perform the slitting and punching operations needed to form the perforation line 22 of the opening flap 24, if this was not already done during fin seal formation. The perforation line 22 may be made by laser cutting or mechanical cutting. Furthermore, when the package is formed from a laminate sheet, the cutting of the line 22 may be through one or more layers.

The finger hole 28 on the opening tab 26 can also be formed at this time. The opening tab 26 and finger hole 28 can be cut out of a sheet material separate from the continuous roll used to create the package. The opening tab 26 and finger hole 28 can be cut simultaneously or separately by a simple punching operation, laser cutting or any other any other way known in the art. Once the opening tab 26 and finger hole 28 are created, the opening tab 26 can be adhered to the front panel 20 in an orientation wherein the finger hole 28 extends beyond the top edge 14 of the package 10 and the remainder of the opening tab overlies the perforation line 22 of the opening flap 24.

The final step in the manufacturing process is severing the finished, leading package off the front end of the sheet material. The severing action separates the side edge seal of the leading package from the side edge seal of a trailing package. Severing leaves a finished package 10 in the condition generally shown in Fig. 1.

To open the package, a user may engage the package 10 in two places, the opening tab 26 (via the finger hole 28 extending beyond the perimeter of the package 10) and anywhere else on the package 10. The user will then open the package 10 by pulling the opening tab down towards the bottom edge 16 of the package 10. As this is done the perforation line 22 will tear to reveal an end of the catheter 12. The user can then easily remove the catheter 12 from the package 10 via the opening flap 24. Using the finger hole 28 on the opening tab 26 that extends beyond the perimeter of the package allows users to more easily grip the opening tab of the package. This contrasts with present packages that are opened by an opening tab that is placed completely within the perimeter of the package.

The flow wrap packaging disclosed herein is one example of a package wherein the opening tab extends beyond the boundary. Such opening tabs may be used with other packages, such as three-sided and four-sided sealed packages that include an opening flap in the front panel.

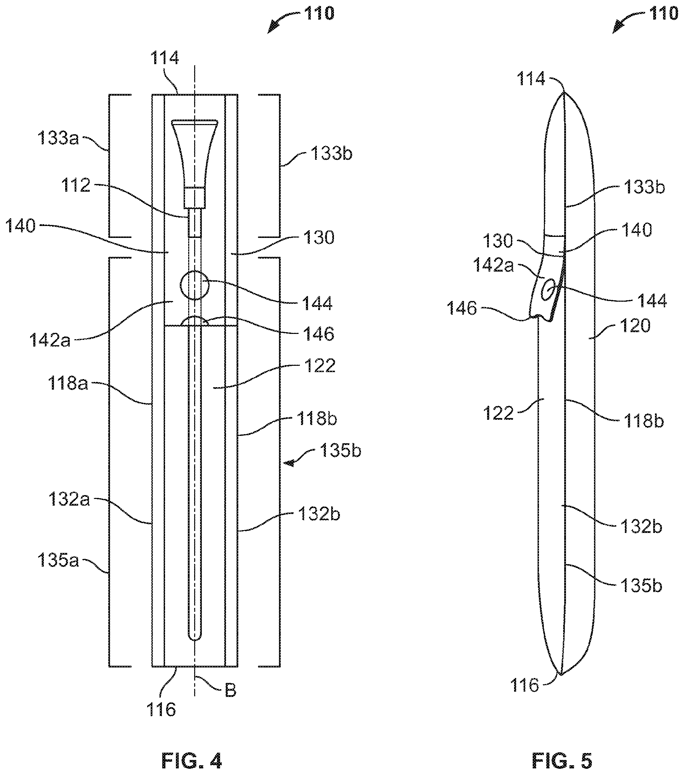

Figs. 4-7 show a package 110 in accordance with the present disclosure. The package 110 is formed by wrapping an elongated sheet material around a catheter 112. Package 110 has many similarities to package 10 shown in Fig. 1 and may be formed by the above described process described in relation to Fig. 3. As will be described in more detail below, package 110 includes a fin seal 130 that extends between the side edge seals and generally perpendicular to the longitudinal axis B of catheter 112. The fin seal 130 may be a peelable seal and opening of the package 110 may be initiated by peeling apart the fin seal 130.

The package includes a top edge 114, bottom edge 116 and opposed side edges 118a and 118b. The package 110 has a front panel 120 and a back panel 122. The package 110 is sealed by a fin seal 130 that is located in the back panel 122 of the package 110. The fin seal 130 is formed by two confronting fin panels of the wrapped sheet material. The fin seal 130 runs perpendicular to the longitudinal axis B of the catheter 112 and across the back panel 122 of the package 110 between side edge seals 132a and 132b.

In the illustrated package 110, the width between edge seals 132a and 132b may be shorter than the length between top edge 114 and bottom edge 115. The side edge seals 132a and 132b are formed by adhering the back panel 122 to the front panel 124 along sheet edges 118a and 118b. The side edge seals 132a and 132b also are peelable seals that may be peel apart to open the package. As shown in Fig. 4, the side edge seals 132a and 132b each may include a top segment 133a and 133b, respectively, and a bottom segment 135a and 135b, respectively. The top and bottom segments may be divided or delineated by the fin seal 130.

As mentioned above, the fin seal 130 may be a peel-apart seal that is peeled apart to initiate opening of the package. Optionally, the fin seal 130 may be offset between the top edge 114 and the bottom edge 116 of the package. That is, the fin seal may be closer to one of the top and bottom edges. In the illustrated embodiment, the fin seal 130 is closer to top edge 114. The fin seal 130 includes a sealed portion 140 and free end portions 142a and 142b that extend from the sealed portion 140. The free end portions 142a and 142b define tabs that may be grasped by the user and pulled apart to open the package 110. The free end portions 142a and 142b may include holes 144 therethrough or cut-outs 146 along the edges that may assist the user in separating and/or grasping the free end portion portions.

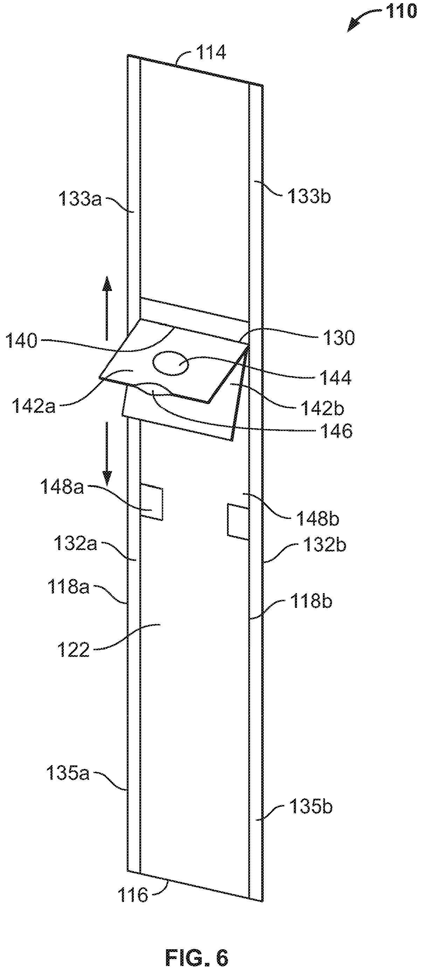

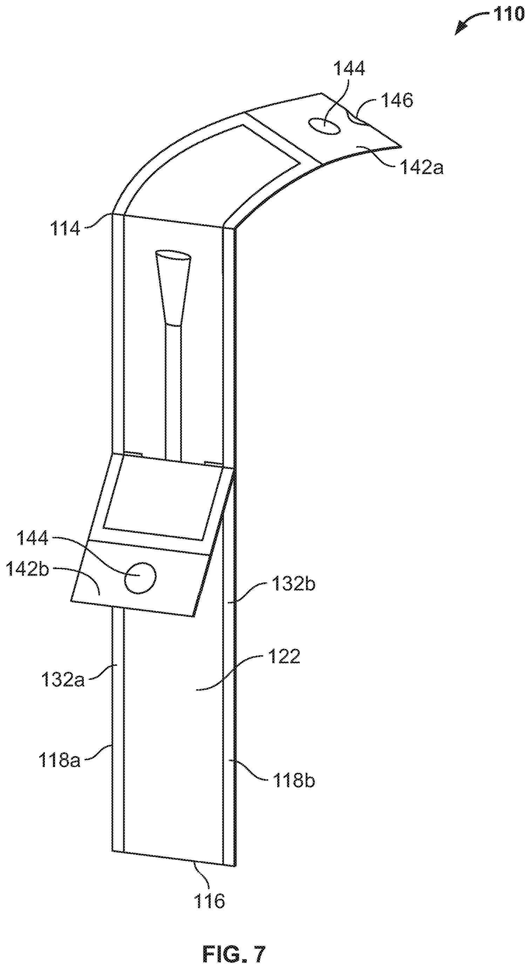

Referring to Figs. 6 and 7, to open the package, the user may grasp one or both of the free end portions/tabs 142a and 142b of fin seal 130 and pull the free end portions 142a and 142b apart. As the user pulls the free end portions apart, the sealed portion 140 of the fin seal 130 peels apart initiating opening of the package. As the user continues to pull the free end portions 142a and 142b apart, the side seals 132a and 132b peel apart to continue opening of the package. For example, as the user pulls free end 142a (which may be a top tab), the top segments 133a and 133b of the side seals 132a and 132b peel apart. The user may peel apart the top segments 133a and 133b of the side seals until a desired size opening is formed. The user may peel apart the top segments 133a and 133b of the side seals all the way to the top edge 114. The user may also pull free end 142b (which may be a bottom tab) to peel the bottom segments 135a and 135b of the side seals 132a and 132b apart. The user may peel apart the bottom segments 135a and 135b of the side seals until a desired size opening is formed. Optionally, the side seals 132a and 132b may include peel stops 148a and 148b (Fig. 6) which prevent or hamper the peeling of the seals such that the user is only able to open the seals a desired amount. The peel stops may include but are not limited to strongly sealed portion adjacent the side seals and/or cut-outs in the side seals.

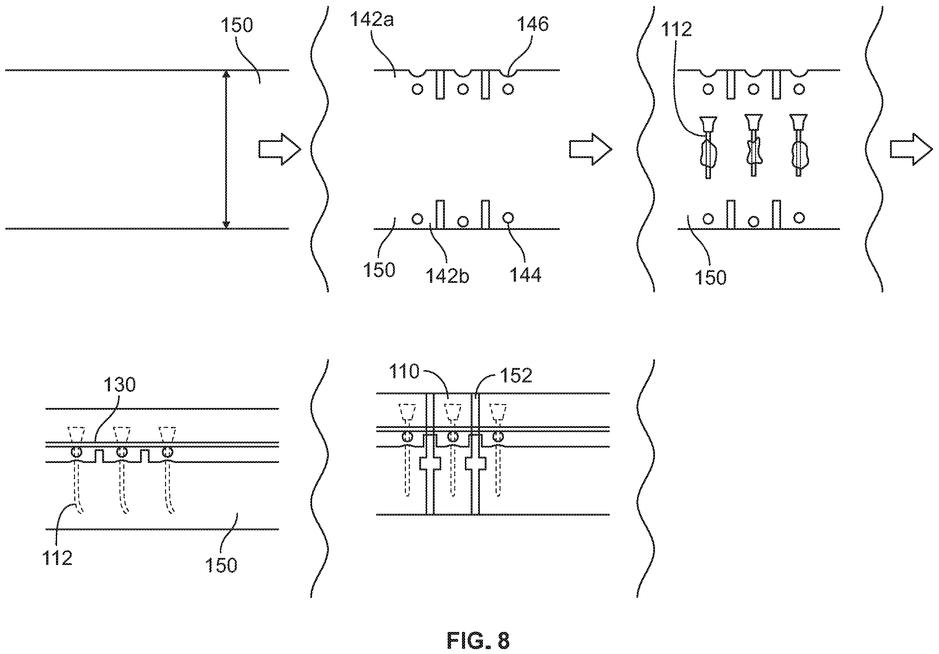

Fig. 8 shows a schematic illustration of one embodiment of a manufacturing process for making a medical product. In step 1, a web 150 of material may be into a flow-wrap apparatus. The web may be a laminate film that includes a polymer and a metal. For example the web may be layers of polyethylene and aluminum. In step 2, the free ends/tabs 142a and 142b, holes 144 and cut-outs 146 of the fin seal 130 may be punched in the web 150. In step 3, the catheter 112 may be placed on the web. In step 4, the web 150 is folded about the catheter 112 and fin seal 130 is formed. In step 6, the side seals are formed and a perforation or cut 152 is made between the individual catheter packages 110.

It should be understood that various changes and modifications to the presently preferred embodiments described herein will be apparent to those skilled in the art. Such changes and modification can be made without departing from the scope of the invention as defined by the appended claims. For example, the arrangement of the opening tab could be other than as shown. The opening tab could extend from anywhere on the front panel 20.

## Claims

1. A catheter product, comprising:
a catheter (12, 112);
a package (10, 110) containing the catheter (12, 112), wherein the package (10, 110) comprises a wrapped elongated sheet material (52) that is wrapped around the catheter, the elongated sheet material (52) having first and second fin forming sections (54a, 54b), first and second back forming sections (56a, 56b), and a front forming section (58); and
the package (10, 110) having a top edge (14, 114), a bottom edge (16, 116) and opposed side edges (18a, 18b, 118a, 118b), the first and second back forming sections (56a, 56b) of the sheet material (52) defining a back panel (122) of the package (10, 110), the front forming section (58) of the sheet material (52) defining a front panel (20, 120) of the package (10, 110), the front and back panels (20, 120, 122) being sealed together to form opposed side seals (32a, 32b, 132a, 132b) that extend along the opposed side edges (18a, 18b, 118a, 118b) of the package (10, 110) and the first and second fin forming sections (54a, 54b) of the sheet material (52) sealed together to form a fin seal (30, 130) that extends between the opposed side seals (32a, 32b, 132a, 132b) and is perpendicular to a longitudinal axis (A, B) of the catheter (12, 112).

2. The catheter product of claim 1 wherein the top edge (14), bottom edge (16) and opposed side edges (18a, 18b) define a perimeter of the package (10) and the front panel (20) includes a perforation line (22) defining an opening flap (24), the package (10) further including an opening tab (26) having a first portion (25) overlying the opening flap (24) attached to the front panel (20) and a second free portion (27) extending beyond the perimeter of the package (10) wherein the free portion (27) provides a gripping portion for opening the package (10).

3. The catheter product of claim 1 wherein the fin seal (130) includes a sealed portion (140) and first and second free end portions (142a, 142b) extending therefrom, the sealed portion (140) being peelable to open the package (110) and the first and second free end portions (142a, 142b) being configured to be pulled apart to peel open the sealed portion (140) of the fin seal (130).

4. The catheter product of claim 1 wherein the fin seal (130) is offset between the top edge (114) and bottom edge (116) of the package (110).

5. The catheter product of any one of claims 3 and 4 wherein the side seals (132a, 132b) are peelable and are configured to be peeled open a selected amount when the package (110) is opened.

6. The catheter product of claim 5 wherein the side seals (132a, 132b) include peel stops (148a, 148b).

7. The catheter product of any one of claims 3-6 wherein the first and/or second free end portions (142a, 142b) includes holes (144) and/or cutouts (146).

## Patentansprüche

1. Katheterprodukt, umfassend:
einen Katheter (12, 112);
ein Gehäuse (10, 110), das den Katheter (12, 112) enthält, wobei das Gehäuse (10, 110) ein gewickeltes, längliches Bahnmaterial (52) umfasst, das um den Katheter gewickelt ist, wobei das längliche Bahnmaterial (52) einen ersten und einen zweiten einen Grat ausbildenden Teilabschnitt (54a, 54b), einen ersten und einen zweiten eine Rückseite ausbildenden Teilabschnitt (56a, 56b) und einen eine Vorderseite ausbildenden Teilabschnitt (58) aufweist; und
wobei das Gehäuse (10, 110) eine Oberkante (14, 114), eine Unterkante (16, 116) und gegenüberliegende Seitenkanten (18a, 18b, 118a, 118b) aufweist, wobei der erste und der zweite eine Rückseite ausbildende Teilabschnitt (56a, 56b) des Bahnmaterials (52) eine Rückwand (122) des Gehäuses (10, 110) definieren, wobei der eine Vorderseite ausbildende Teilabschnitt (58) des Bahnmaterials (52) eine Frontwand (20, 120) des Gehäuses (10, 110) definiert, wobei die Front- und Rückwand (20, 120, 122) miteinander versiegelt sind, um gegenüberliegende Seitenversiegelungen (32a, 32b, 132a, 132b) auszubilden, die sich entlang der gegenüberliegenden Seitenkanten (18a, 18b, 118a, 118b) des Gehäuses (10, 110) erstrecken, und der erste und zweite einen Grat ausbildende Teilabschnitt (54a, 54b) des Bahnmaterials (52) miteinander versiegelt sind, um eine Gratversiegelung (30, 130) auszubilden, die sich zwischen den gegenüberliegenden Seitenversiegelungen (32a, 32b, 132a, 132b) erstreckt und senkrecht zu einer Längsachse (A, B) des Katheters (12, 112) verläuft.

2. Katheterprodukt nach Anspruch 1, wobei die Oberkante (14), die Unterkante (16) und die gegenüberliegenden Seitenkanten (18a, 18b) einen Umfang des Gehäuses (10) definieren und die Frontwand (20) eine Perforationslinie (22) beinhaltet, die eine Öffnungsklappe (24) definiert, wobei das Gehäuse (10) ferner eine Öffnungslasche (26) beinhaltet, die einen ersten Abschnitt (25), der die Öffnungsklappe (24) überlappt und an der Frontwand (20) befestigt ist, und einen zweiten freien Abschnitt (27) aufweist, der sich über den Umfang des Gehäuses (10) hinaus erstreckt, wobei der freie Abschnitt (27) einen Griffabschnitt zum Öffnen des Gehäuses (10) bereitstellt.

3. Katheterprodukt nach Anspruch 1, wobei die Gratversiegelung (130) einen versiegelten Abschnitt (140) und einen ersten und einen zweiten freien Endabschnitt (142a, 142b) beinhaltet, die sich von diesem erstrecken, wobei der versiegelte Abschnitt (140) abziehbar ist, um das Gehäuse (110) zu öffnen, und der erste und der zweite freie Endabschnitt (142a, 142b) konfiguriert sind, um auseinandergezogen zu werden, um den versiegelten Abschnitt (140) der Gratversiegelung (130) aufzuziehen.

4. Katheterprodukt nach Anspruch 1, wobei die Gratversiegelung (130) zwischen der Oberkante (114) und der Unterkante (116) des Gehäuses (110) versetzt ist.

5. Katheterprodukt nach einem der Ansprüche 3 oder 4, wobei die Seitenversiegelungen (132a, 132b) abziehbar und konfiguriert sind, um beim Öffnen des Gehäuses (110) um ein ausgewähltes Ausmaß aufgezogen zu werden.

6. Katheterprodukt nach Anspruch 5, wobei die Seitenversiegelungen (132a, 132b) Abziehsperren (148a, 148b) beinhalten.

7. Katheterprodukt nach einem der Ansprüche 3 bis 6, wobei der erste und/oder der zweite freie Endabschnitt (142a, 142b) Löcher (144) und/oder Aussparungen (146) beinhaltet/beinhalten.

## Revendications

1. Produit de cathéter, comprenant :
un cathéter (12, 112) ;
un emballage (10, 110) contenant le cathéter (12, 112), dans lequel l'emballage (10, 110) comprend un matériau en feuille allongé enroulé (52) qui est enroulé autour du cathéter, le matériau en feuille allongé (52) présentant des première et seconde sections de formation d'aileron (54a, 54b), des première et seconde sections de formation arrière (56a, 56b), et une section de formation avant (58) ; et
l'emballage (10, 110) présentant un bord supérieur (14, 114), un bord inférieur (16, 116) et des bords latéraux opposés (18a, 18b, 118a, 118b), les première et seconde sections de formation arrière (56a, 56b) du matériau en feuille (52) définissant un panneau arrière (122) de l'emballage (10, 110), la section de formation avant (58) du matériau en feuille (52) définissant un panneau avant (20, 120) de l'emballage (10, 110), les panneaux avant et arrière (20, 120, 122) étant scellés ensemble pour former des joints d'étanchéité latéraux opposés (32a, 32b, 132a, 132b) qui s'étendent le long des bords latéraux opposés (18a, 18b, 118a, 118b) de l'emballage (10, 110), et les première et seconde sections de formation d'aileron (54a, 54b) du matériau en feuille (52) étant scellées ensemble pour former un joint d'étanchéité en aileron (30, 130) qui s'étend entre les joints d'étanchéité latéraux opposés (32a, 32b, 132a, 132b) et est perpendiculaire à un axe longitudinal (A, B) du cathéter (12, 112).

2. Produit de cathéter selon la revendication 1, dans lequel le bord supérieur (14), le bord inférieur (16) et les bords latéraux opposés (18a, 18b) définissent un périmètre de l'emballage (10) et le panneau avant (20) comporte une ligne de perforation (22) définissant un rabat d'ouverture (24), l'emballage (10) comprenant en outre une languette d'ouverture (26) présentant une première partie (25) recouvrant le rabat d'ouverture (24) fixée au panneau avant (20) et une seconde partie libre (27) s'étendant au-delà du périmètre de l'emballage (10), dans lequel la seconde partie libre (27) fournit une partie de préhension pour ouvrir l'emballage (10).

3. Produit de cathéter selon la revendication 1, dans lequel le joint d'étanchéité en aileron (130) comporte une partie scellée (140) et des première et seconde parties d'extrémité libre (142a, 142b) s'étendant depuis celle-ci, la partie scellée (140) étant pelable pour ouvrir l'emballage (110) et les première et seconde parties d'extrémité libre (142a, 142b) étant configurées pour être écartées l'une de l'autre afin d'ouvrir par pelage la partie scellée (140) du joint d'étanchéité en aileron (130).

4. Produit de cathéter selon la revendication 1, dans lequel le joint d'étanchéité en aileron (130) est décalé entre le bord supérieur (114) et le bord inférieur (116) de l'emballage (110).

5. Produit de cathéter selon l'une quelconque des revendications 3 et 4, dans lequel les joints d'étanchéité latéraux (132a, 132b) sont pelables et sont configurés pour être ouverts par pelage d'une quantité sélectionnée lorsque l'emballage (110) est ouvert.

6. Produit de cathéter selon la revendication 5, dans lequel les joints d'étanchéité latéraux (132a, 132b) comprennent des butées de pelage (148a, 148b).

7. Produit de cathéter selon l'une quelconque des revendications 3 à 6, dans lequel les première et/ou seconde parties d'extrémité libre (142a, 142b) comportent des trous (144) et/ou des découpes (146).
